(19) **Europäisches Patentamt / European Patent Office / Office européen des brevets**

(11) **EP 3 407 953 B1**

(12) **EUROPEAN PATENT SPECIFICATION**

(45) Date of publication and mention
of the grant of the patent:
**15.03.2023 Bulletin 2023/11**

(21) Application number: **16888344.5**

(22) Date of filing: **25.01.2016**

(51) International Patent Classification (IPC):
**A61M 16/06** *(2006.01)* **A62B 18/02** *(2006.01)*

(52) Cooperative Patent Classification (CPC):
(C-Sets available)
**A61M 16/06; A61M 16/1065; A61M 16/208;**
A61M 16/0677; A61M 2202/0208; A61M 2202/0225;
A61M 2205/05; A61M 2205/7518; A61M 2230/432

(Cont.)

(86) International application number:
**PCT/US2016/014671**

(87) International publication number:
**WO 2017/131607 (03.08.2017 Gazette 2017/31)**

(54) **OXYGEN MASKS**

SAUERSTOFFMASKEN

MASQUES À OXYGÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.12.2018 Bulletin 2018/49**

(73) Proprietor: **Chang, Alexander
Los Angeles CA 90013 (US)**

(72) Inventor: **Chang, Alexander
Los Angeles CA 90013 (US)**

(74) Representative: **HGF
HGF BV
Benoordenhoutseweg 46
2596 BC The Hague (NL)**

(56) References cited:
| | |
|---|---|
| WO-A1-99/61090 | WO-A1-2010/067235 |
| WO-A1-2014/124323 | CN-A- 104 587 580 |
| US-A1- 2003 024 533 | US-A1- 2012 216 806 |
| US-A1- 2012 216 806 | US-A1- 2015 119 742 |
| US-A1- 2015 217 075 | US-A1- 2016 030 695 |
| US-B2- 6 792 943 | US-B2- 7 004 168 |

(52) Cooperative Patent Classification (CPC): (Cont.)

    C-Sets
    A61M 2202/0208, A61M 2202/0007;
    A61M 2202/0225, A61M 2202/0085

**Description**

FIELD

The present disclosure relates to masks for facilitating the delivery oxygen to a patient. More particularly, it relates to an oxygen mask operative to define an oxygen reservoir that collects and stores the amount of oxygen available for a patient to breathe in while undergoing medical procedures.

BACKGROUND

This section provides background information related to the present disclosure which is not necessarily prior art.

Oxygen masks are connected to a source of oxygen that is delivered directly to the patient's airway, namely, the nose and mouth. Conventional oxygen masks generally cover the entire nose and mouth areas and have no apertures to allow surgical tools to access the mouth or nose. Notwithstanding the ability of conventional masks to form a seal about the patient's airway, and attempt to confine the area about the airway to which oxygen is delivered, conventional masks suffer from numerous drawbacks. In particular, most conventional oxygen masks have a tendency to dilute the oxygen being delivered to the patient. Also, those masks cause oxygen to be dispersed about and away from the patient's airway, which can lead to ineffective oxygen delivery.

Nasal cannulation can be used as primary and only source of oxygenation device. This approach has problems. For example, nasal cannulation delivers oxygen typically ineffectively and wastefully. The structure and function of most conventional oxygen masks assume that the patient undergoing the procedure will breathe in oxygen precisely as delivered. Additionally, this approach assumes that infusing sufficiently large volumes of oxygen will, in fact, ultimately reach the patient. The logic is flawed. Due to the dilution and dispersion of the oxygen being delivered by conventional masks, this practice is extremely wasteful. Attempting to deliver oxygen in the aforementioned manner wastes considerable amounts of oxygen that must be constantly delivered throughout the entire respiratory cycle. Only a very small portion of the oxygen is delivered to the patient.

In an attempt to address the foregoing issues, a specific type of oxygen mask/delivery system has been developed that essentially deploys a "face tent". After the patient assumes the lateral decubitus or prone position, a plastic sheet or bag is used to cover the patient's face, following nasal cannulation. The plastic sheet or bag is operative to define a tent over the airway that maintains a reservoir of oxygen supposedly made available to the patient. These modified oxygen masks, often referred to as the TSE mask, has been discussed in Shaul Cohen, et al., "TSE 'Mask' Improves Oxygenation in Deeply Sedated Patients with Nasal Cannula during Upper Endoscopy." Anesthesiology 107:A922, 2007. Poster Presentation at American Society of Anesthesiologists Annual Meeting, October 2007, Chicago, IL.

The TSE mask is designed to improve effectiveness at inhibiting oxygen desaturation. First, the oxygen reservoir of the TSE mask can provide an inspiratory fraction of oxygen of 40-60% with oxygen flows of 4 L/min. Accordingly, titrating intravenous sedatives after pre-oxygenation can achieve moderate-to-deep sedation while maintaining spontaneous respiration without oxygen desaturation. Second, the patient's respirations are monitored with capnography or a pediatric precordial stethoscope placed over the trachea. Thus, if the patient becomes apneic because of airway obstruction or over-sedation, medical personnel still have an average of two to three minutes to manipulate the airway before oxygen desaturation occurs.

Additionally, as discussed in Leigh JM, "Variation in Performance of Oxygen Therapy Devices." Annals of The Royal College of Surgeons of England. 1973;52(4):234-253, oxygen therapy has always lacked control over dosage of oxygen. This lack of control over dosage of oxygen applies to conventional oxygen masks that are used for oxygen therapy. As stated in Leigh, the use of conventional oxygen masks "still rarely conforms to the most elementary rational rules of therapeutics - that is, that a substance is given in a known dosage in a manner which produces a sustained optimal blood level." The inability to consistently deliver a known dosage of oxygen is caused at least in part by the difficulty in assessing the patient-device interrelationship. Patient factors, such as peak inspiratory flow rate ("PIFR") can affect the amount of oxygen that is delivered to the patient. For example, the PIFR can affect the mixture of oxygen, expired gases, and room air in the oxygen mask. Thus, the patient factor PIFR can result in unknown amounts of oxygen being inhaled by the patient on each breath.

Ventimask® is a conventional oxygen mask designed to improve effectiveness at controlling the dosage of oxygen delivered to the patient. The Ventimask attempts to achieve this improved control by reducing the dependency of oxygen dosage on the patient factor PIFR. To do so, the Ventimask is a high-flow device that delivers oxygen mixtures at flows above the PIFR (such as at 30L/min - 45 L/min). However, in order to reduce dependency on PIFR, the Ventimask is limited to high-flow rates, specifically flow rates that are greater than or equal to the PIFR.

Additionally, conventional masks routinely suffer in performance because of sealing issues. Medical personal commonly do not take extra caution in sealing conventional oxygen masks to the face of patients, causing leaks and allowing room air entrainment. The room air entrainment exasperates the problem of conventional oxygen masks lacking control over the dosage of oxygen as oxygen is further diluted with room air. Even in cases that conventional oxygen masks are carefully applied to the patients' faces, leaks commonly exist because of the differences between the contours of the conventional oxygen masks and the shape of the patients' faces. Thus, leaks caused by incomplete seals are a common factor contributing to the lack of control of oxygen dosage of conventional oxygen masks. The oxygen mask described in US7004168 is designed also to address this issue.

Furthermore, conventional masks are typically ill-suited for use in upper endoscopy procedures that require the surgical instrumentation be deployed through the mouth and throat of the patient. For example, conventional masks do not include a sufficient aperture to allow surgical tools to access the mouth or nose. In this regard, the oxygen mask needs to effectively deliver oxygen, while having an opening or orifice through which surgical instrumentation can be deployed. The opening or orifice in the mask must have enough flexibility to enable the instrumentation to be easily and readily manipulated, as occurs when instrumentation is deployed in upper endoscopy procedures.

In attempt to introduce an oxygen mask suitable for allowing surgical tools to access the mouth and nose during upper endoscopy procedures, a specific type of oxygen mask/delivery system has been developed. These surgical masks, often referred to as the Panoramic Oxygen Mask ("POM") has been discussed in US 8,960,195 to Lehman. US 8,960,195 describes an intubation-facilitating oxygen mask that can be worn during standard oxygen therapy. This mask is intended to provide for increased oxygen saturation and continuous monitoring of carbon dioxide of expired gases. This mask introduces an aperture in the mask above the mouth to allow surgical tools to access the mouth and nose during upper endoscopy procedures.

Although some conventional masks are designed for upper endoscopy procedures, these conventional masks still present health risks. For example, conventional masks designed for upper endoscopy procedures provide sub-optimal monitoring of patients. In 80% or more cases when patients are sedated, an anesthesiologist may not be present in the procedure room. In such situations, nurses generally monitor the patient for oxygenation status and breathing status in addition to their normal workload (e.g., paper work, administration of medication, following orders from endoscopists or other physicians, securing agitated and/or thrashing patients). Indications of breathing can include: level of consciousness (e.g., spoken response to commands can determine the patient's level of consciousness), visual breathing signs, vital signs (e.g., blood pressure, heart rate), EKG, oxygen saturation (oximetry), and $CO_2$ levels. However, determining all of these indications can occur in dark conditions or settings with less light, which can reduce accurate monitoring of patient breathing and oxygenation statuses.

Most conventional and common method of oxygen supplement for upper endoscopy use nasal cannula and an end tidal $CO_2$ machine is typically used to monitor breathing. To measure the oxygen condition of the patient, a finger monitor is typically used. However, these monitoring techniques are problematic. For example, the $CO_2$ monitoring machine uses the same or similar alarm when a negative occurrence is detected. Therefore, the alarm sound of the $CO_2$ monitoring machine will not specify to the caretaker whether the patient is breathing, $O_2$ saturation is low, or any other number of reasons that trigger the alarm. Further, the $CO_2$ monitoring machine does not indicate what caused the trigger. For example, if $O_2$ saturation is low, the caretaker still has to determine whether it was because the patient is breathing through the mouth or for some other reason. Each reason that can trigger the alarm can have a different appropriate response. If the patient has too little oxygen in the body, then the $O_2$ flow rate can be increased to provide more oxygen. If the patient's airway is obstructed, then the airway must be opened. Without knowing the cause of the alarm, caretakers must take time to identify the cause. In many cases, false alarms can be triggered by events such as the patient thrashing, further making it harder for caretakers to respond appropriately and disturbing the working environment. The net effect of the $CO_2$ monitoring techniques of conventional oxygen masks designed for upper endoscopy procedures is compromised patient safety.

Despite various attempts to improve upon other conventional masks, all conventional masks (including masks designed for upper endoscopy procedures) have one or more of the following safety and effectiveness related issues: air entrainment, re-breathing of mechanical dead space air, no effective sequential oxygen delivery system, wasting of oxygen (or other target gas or drug), limited in dosage control, ineffective monitoring of expired gases for early recognition of undesired events, nuisance of false alarms, bulkiness, and high cost.

It has been recognized that there is a substantial need for an oxygen mask that can more efficiently and effectively provide oxygen enriched gas to be more readily taken in by a patient's airway while the patient breathes while wearing the oxygen mask.

There is a further need for such an oxygen mask that can improve monitoring of patients undergoing upper endoscopy procedures for early detection and prevention of oxygen desaturation or other undesired events.

There is a further need for such an oxygen mask that can make available a reservoir of oxygen enriched air immediately adjacent the patient's airway upon the uptake of a breath by the patient to a degree greater than conventional oxygen masks.

Moreover, there is a need for such a mask that can continuously maintain an enriched source of oxygen as maintained in a reservoir immediately accessible to the patient's airway while at the same time consuming substantially less amounts of oxygen than prior art oxygen masks.

There is still a further need for such an oxygen mask that is of simple construction, easy to deploy, can be readily used with nasal cannulation as a combined synergistic device, and can be utilized on patients undergoing upper endoscopy procedures such that instrumentation can be deployed through such mask with relative ease and flexibility.

SUMMARY

This section provides a general summary of the disclosure and is not a comprehensive disclosure of its full scope or all of its features. The scope of the present invention is defined by the appended claims.

In one embodiment, not being part of the invention, there is provided an oxygen mask comprising: a mask body defining a cavity configured to be positioned over the mouth and nose of a patient, an oxygen port formed on the upper half of the mask body, an annular aperture formed on the mask body, and at least one vent port formed on the mask body, wherein each vent port is formed on the bottom half of the mask body in a manner that patient's exhaled gases are directed towards the vent port.

According to the present invention, there is provided an oxygen mask comprising: a mask body defining a cavity configured to be positioned over the mouth and nose of a patient, an oxygen port formed on the upper half of the mask body, an oxygen storage tube directly coupled to the bottom half of the mask body, an annular aperture formed on the mask body, and a vent port formed on the oxygen storage tube, wherein the vent port is formed on the oxygen storage tube in a manner that patient's exhaled gases are directed towards the vent port.

In yet another embodiment, not being part of the present invention, there is provided an oxygen mask comprising: a mask body defining a cavity configured to be positioned over the nose of a patient without covering the mouth of the patient, an oxygen port formed on about the middle or the upper half of the mask body, an oxygen storage tube directly coupled to the bottom half of the mask body, a vent port formed on the oxygen storage tube, wherein the vent port is formed on the oxygen storage tube in a manner that patient's exhaled gases are directed towards the vent port.

In yet another embodiment, not being part of the present invention, there is provided a nasal pillow oxygen mask comprising: a mask body configured to be positioned near the nose of a patient without covering the mouth of the patient, one or two nasal pillow plugs configured to be connected to an oxygen source, an oxygen port, a reservoir tube directly coupled to the bottom half of the mask body, a vent port formed on the oxygen reservoir tube, wherein the vent port is formed on the oxygen reservoir tube in a manner that patient's exhaled gases are directed towards the vent port.

Further areas of applicability will become apparent from the description provided herein.

BRIEF DESCRIPTION OF THE DRAWINGS

Fig. 1 shows a conventional simple face mask.
Fig. 2 shows an embodiment of a low $FiO_2$ mask in accordance with the present disclosure.
Fig. 3 illustrates comparison of a conventional mask, such as Fig. 1, and an embodiment of Fig. 2.
Fig. 4 shows the mask of Fig. 2 further comprising filters.
Fig. 5 shows an embodiment of a high $FiO_2$ mask in accordance with the present invention.
Fig. 6 shows a nasal oxygen conserving mask in accordance with the present disclosure.
Fig. 7 shows an embodiment of an endoscopy mask in accordance with the present disclosure.
Fig. 8 shows the endoscopy mask of Fig. 7 further comprising recesses to allow nasal cannula to directly access the inner-space.

DETAILED DESCRIPTION

A multi-functional oxygen mask is provided for improving and controlling alveolar oxygen concentration. This can be achieved by strategically supplying oxygen in accordance to the respiratory cycle, which consists of three phases: expiration, expiratory pause (i.e., post expiration pause), and inspiration.

Conventional oxygen masks result in dilution of the intended oxygen (or other gas or drug) with significant amounts of room air during each inspiration, especially the early part of inspiration. In some instances, the intended therapy (e.g., oxygen, other gas, and drug) can be effectively reduced by half or more. This dilution occurs around the nasal area if a nasal cannula is utilized and around the vent ports located near the nasal area if an open or vented system mask such as a simple face mask or non-rebreathing mask is used as the supplemental oxygen delivery device. Thus, patients do not receive the required or appropriate amount of oxygen or drug.

The oxygen mask of the present disclosure can provide at least the following benefits:

1. Improved rescue capability during sedated medical procedures:

By increasing the oxygen reserves in the patient body during sedated upper endoscopic procedures, the oxygen mask of the present disclosure can prolong the safe period of apnea time from airway obstruction. Thus, caretakers will have extra time to resolve the airway problem. This extra time can be life-saving.

Additionally, the oxygen mask of the present disclosure provides a more specific and sensitive $CO_2$ monitor that can result in fewer false alarms compared to vented open face masks or nasal cannula. The higher accuracy in detecting respiratory problems can allow better and earlier detection of problems. As is known, capnograms (wave form) are very important and must be deployed when a patient is under sedation for upper endoscope procedures. Capnograms tell whether a patient is breathing or not, and if there is no $CO_2$ waveform, it means there is no $CO_2$ on breathing (expiration). This usually means that there is an airway obstruction or that the patient is not breathing or may be over-sedated or that the heart is not pumping (i.e., cardiac arrest). It also indicates a slowdown in respiration due to over-sedation. It is the earliest detectable sign for highly undesirable events and an earlier signal than pulse oximeter.

The oxygen mask of the present disclosure can lessen dilution or dispersion of $CO_2$ compared to conventional masks (e.g., open airway masks) or nasal cannula alone. No (or substantially no) $CO_2$ accumulates on each breath with the oxygen mask of the present disclosure because $CO_2$ is pushed out of the oxygen mask by continuous oxygen flow during expiratory pause. Thus, there is no (or substantially no) $CO_2$ present during inspiration, and expired $CO_2$ will be less diluted and less dispersed during expiration because the oxygen mask of the present disclosure requires a lower continuous oxygen flow. Since $CO_2$ is less diluted and dispersed, $CO_2$ monitoring is more specific for respiratory problems. For example, the oxygen mask of the present disclosure can help customize and tailor $CO_2$ monitoring to fit each patient's specific needs on every breath.

By providing a mechanism that enables help to prevent or correct a obstruction of the patient's air way to be more quickly and clearly identified, the oxygen mask of the present disclosure can enable caretakers to more promptly take appropriate action. For example, if a patient becomes apneic because of airway obstruction or over-sedation, the care team can still have a few to several minutes to manipulate the airway before oxygen desaturation occurs. This is due to the high efficacy of alveolar oxygenation and early detection of airway obstruction by the oxygen mask of the present disclosure, which prolongs the safe period of apnea time.

2. Reduction of the number of misdiagnoses of medical conditions:

Conventional oxygen masks can lead to misdiagnosis or false alarms of medical conditions due to various problems (e.g., base line elevating due to rebreathing, less $CO_2$ due to dilution). These flawed masks cause false alarms which interfere with the work flow and there is a tendency to ignore most important monitoring. For example, an ineffective and inefficient oxygen and/or drug delivery system (e.g., because of room air dilution) can lead to a misleading value of the amount of oxygen the patient receives. In doing so, there can be an impression of a worse cardiopulmonary shunt than actually exists, potentiating the need for unnecessary invasive and expensive diagnostic procedures and therapies. However, by reducing or eliminating dilution of oxygen (or other intended therapy), the oxygen mask of the present disclosure can reduce faulty estimations of oxygen delivered to patients. This in turn can reduce misdiagnoses of medical conditions, thereby preventing unnecessary medical procedures which are sometimes risky and costly, for example, such as an invasive medical interventions or an endotracheal intubation requiring Intensive Care Unit services.

3. Reduction or elimination of rebreathing of exhaled gases:

During expiration, fresh continuous oxygen flow pushes away exhaled gas (e.g., dead space gas, carbon dioxide) and any room air that may have entered the oxygen mask. These gases are pushed into the atmosphere through vent ports that are formed on the bottom half of the oxygen mask. After each expiration, there is an expiratory pause lasting approximately 1/3 of a respiratory cycle, during which no respiratory (expiration) air will flow. The fresh continuous oxygen flow continues to push away exhaled gas from the upper half of the oxygen mask to the bottom half. Exhaled gases and any room air that entered the oxygen mask are pushed out from the reservoir into the atmosphere until all or almost all exhaled gases are pushed out into the atmosphere. This function of the oxygen mask of the present disclosure can eliminate variability related to the patient's expiration time which can affect the gas component of the reservoir (e.g., $CO_2$) for the next inspiration.

While pushing away the exhaled gas and any room air inside the oxygen mask, the fresh continuous oxygen fills a reservoir-like space inside of the oxygen mask between the nose area and vent port. In this manner, near 100% oxygen is collected and stored near the nose for immediate intake during the next inspiration. Thus, on inspiration, the patient inhales 100% or near 100% oxygen. Accordingly, hypercarbia is inhibited by virtue of no or insubstantial carbon dioxide being inhaled.

4. Reduction or elimination of oxygen dilution with room air:

With conventional oxygen delivery devices, there is sudden make up tidal volume during each inspiration. This is especially true during the early part of inspiration when the peak inspiratory flow rate ("PIFR") occurs. With conventional oxygen delivery devices, a significant amount of room air flows into nose and dilutes the supplied oxygen during the PIFR time. This dilution occurs around the nasal area if a nasal cannula is utilized. If a vented oxygen mask is used, two (or sometimes one) ventilation ports on each side of mask near the nose area are responsible for the dilution during the active early phase of inspiration. Because of this early inspiration phenomenon, open or vented oxygen delivery system devices, such as the simple face mask, non-rebreather mask (i.e., a one-way valve removed mask), have significant air dilution effects on alveolar oxygenation. Also, the Venturi mask and high flow nasal cannula ("HFNC"), require high gas flows to avoid the effect of room air dilution from PIFR effect. These devices can thus require 35 to 45 L/minute of gas flow to overcome room air entrainment for normal spontaneous respiration averaging 3 to 4 times of minute ventilation.

To avoid room air dilution, the oxygen mask of the present disclosure places each vent port to opposite top-or-bottom half from nasal area to create a gas reservoir between the nasal area and vent port(s). During each early part of inspiration, 100% oxygen that is stored during expiratory pause is breathed into the lungs first instead of diluted room air from the vent port(s), thereby avoiding (or delaying) PIFR effect with room air. As such, the oxygen mask of the present disclosure can deliver stored amount of known 100% (or near 100%) oxygen in the reservoir without room air dilution prior to entering the lungs.

The basic physiological function of the PIFR is to enhance the velocity of air movement towards the alveolar to maximize effective and efficient oxygenation especially at the very early part of inspiration during normal spontaneous breathing. Thus, any air dilution at the early part of inspiration with conventional oxygen devices can cause a lower fraction of inspired $O_2$, which runs against the purpose of the PIFR seen during spontaneous breathing. The oxygen mask of the present disclosure can maximize the effect of the PIFR of any target gas or drug needed to be delivered from the reservoir of the oxygen mask of the present disclosure to the lungs by delivering a known amount or dosage of oxygen (or drug or other gas) to patients on controlled oxygen therapy.

5. Reduction of oxygen wasting:

Conventional oxygen masks do not fully appreciate that the first half of inspiration is responsible (either fully or mostly) for alveolar oxygenation. It has been clinically observed that oxygen is absorbed into the blood essentially only during an early stage of inspiration in the breathing process. That is, it is during an early stage of inspiration that oxygen effectively reaches the alveoli. Oxygen applied during the latter stages of inspiration remains in "dead spaces" such as the pharynx, trachea, and bronchial tubes. Thus, approximately 1/6th of the respiratory cycle is critical for alveolar oxygenation (early part of first half of inspiration). Hence, it has been observed and concluded that it is more advantageous to apply a greater volume of oxygen. Also, it is useful (and effective) to apply the oxygen only during an effective early stage of inspiration.

The effective early stage or peak inspiration flow rate time may only last for less than a second. For example, the effective early stage may last for approximately 0.2 to 0.3 seconds. In another example, the effective early stage may last for approximately 0.25 seconds. For most cases, the effective early stage is less than approximately one-quarter and usually approximately one-eighth of the duration of inspiration. Therefore, if oxygen were supplied at twice the normal volume per second rate (for example, 100 cc/s rather than 50 cc/s), a savings of more than one-half would be realized. In some cases, a savings of more than three-quarters would be realized.

Conventional oxygen masks are not capable of operating in accordance with this effective early stage inspiratory phenomenon. Thus, conventional oxygen masks are incapable of delivering 100% or close to 100% oxygen during the early stage of the first half of inspiration. Without any means for storing oxygen during expiratory pause, conventional oxygen masks waste oxygen that is continuous flowing (by sending the oxygen into the atmosphere) during these phases. Additionally, even during the first half of inspiration, much of the oxygen can be diluted with atmospheric air resulting in oxygen being wasted and/or being in low concentrations from the effect of the peak inspiration flow rate even during this most efficient phase. In some instances, less than 10% of continuous flowing oxygen is delivered to the alveolar during each breath.

The oxygen mask of the present disclosure can reduce wasting oxygen in at least two ways. First, by removing all or almost all non-oxygen gases (e.g., $CO_2$ and nitrogen) from the oxygen mask between expiration and the end of expiratory pause, there may be no or insignificant air entrainment. Therefore, 100% (or near 100%) oxygen can be delivered to the alveolar during the early part of the first half of inspiration. Second, the oxygen mask of the present disclosure provides a reservoir in the oxygen mask to collect and/or store oxygen during expiratory pause. In doing so, continuously flowing oxygen can be retained even while the patient is not inhaling. Accordingly, wasting of fresh oxygen is reduced.

6. Reduce or eliminate room air entrainment effects from improper mask sealing:

The oxygen mask of the present disclosure can reduce or eliminate room air entrainment effects from improper mask sealing. While room air entrainment can occur through the vent ports of conventional masks, room air entrainment can also occur from improper mask sealing (e.g., gaps between mask and patient's face). In certain circumstances, medical personal may be inattentive to proper mask sealing as the room air entrainment effects of improper mask sealing is generally overshadowed by the room air entrainment effects of vent ports. In situations when the medical personal is inattentive, the oxygen mask of the present disclosure may not be fully secured and sealed to the patient's face, causing leaks. While conventional oxygen masks are inclined to further lack control of oxygen dosage because of room air entrainment caused by such leaks, the oxygen mask of the present disclosure can reduce or eliminate the effects of room air entrainment caused by improper mask sealing. By pushing any expired gases and room air out of the mask during expiration and by storing a bolus of 100% oxygen near the nasal area during expiration and expiratory pause, the patient receives 100% of near 100% oxygen at least at the first half of inspiration. Thus, the oxygen mask of the present disclosure reduces or eliminates the effect of room air entrainment caused by improper mask sealing on the control of dosage of oxygen.

7. Improvement of dosage control:

The following properties can allow the oxygen mask of the present disclosure to improve dosage control:

1) No (or substantially no) air entrainment (no dilution with room air) during early inspiration; Eliminate or reduce patient and the device variabilities for the room air entrainment from the effect of PIFR at the early inspiration;
2) No $CO_2$ re-breathing; Eliminate or reduce patient and device variabilities on expiration time;
3) Saving of known quantity of 100% oxygen in the reservoir during every pause;
4) Known quantity of 100% (or substantially 100%) oxygen first delivered to the alveolar via the Sequential Gas Delivery ("SGD") system, followed by known quantity of 100% (or substantially 100%) continuous flowing oxygen with 21% oxygen with known quantity of room air during first half of inspiration; and
5) Ability to control oxygen amount (dosage) by adjusting the reservoir volume of the mask and oxygen flow rate.

Conventional oxygen delivery devices rely on the promised accuracy of $FiO_2$ at the device level, not the alveolar level. The oxygen mask of the present disclosure can deliver, control, titrate, and maintain the desired proper oxygen amount (dosage) at the alveolar level.

The volume of the reservoir can vary depending on the desired $O_2$ concentration. In some cases, the target $FiO_2$ can be from about 24% to about 60%. In other cases, the target $FiO_2$ can be from about 50% to about 100%. According to another embodiment, the reservoir varies from approximately 25 cc to 150 cc and the oxygen mask can store a total of up to approximately 400 cc via reservoir and attached oxygen storage reservoir tube. The storage reservoir volume and oxygen flow rate of the oxygen mask of the present disclosure can be adjusted to accommodate each patient's needs.

For example, the necessary storage reservoir volume and flow rate can be calculated using the patient tidal volume ("TV"), respiratory rate ("RR"), and target $FiO_2$. If the patient TV is 450 cc, respiratory rate is 20/min, and target $FiO_2$ is 100%, then the storage reservoir volume and oxygen flow rate can be calculated as follows:

Since 2/3 of the patient TV is delivered to the alveolar in the first half of inspiration, 300 cc (2/3 x 450 cc = 300 cc) is delivered to the alveolar. Using the oxygen mask of the present disclosure, the 300 cc delivered to the alveolar in the first half of inspiration can be 100% (or substantially 100%) $FiO_2$. Thus, if "X" represents the amount of 100% oxygen stored in the reservoir during expiratory pause, which constitutes 1/3 of the breathing cycle, then ½X represents the amount of oxygen delivered from the continuously flowing oxygen source during the first half of inspiration (as the first half of inspiration is ½ the time duration of expiration). If "RA" represents the amount of 100% oxygen in the room air, which is 21% oxygen, during the first half of inspiration, then the sum of X, ½X, and RA is the final volume delivered to the alveolar in the first half of inspiration (300 cc). This is shown in equation format as: X + ½X + RA = 300 cc of 100% oxygen. Solving for X shows what the total reservoir volume of the oxygen mask of the present disclosure should be given a 300 cc final volume delivered to the alveolar in the first half of inspiration:

$$X + \tfrac{1}{2}X + RA = 300 \text{ cc of } 100\% \text{ oxygen}$$

$$X + \tfrac{1}{2}X = 300 \text{ cc - RA}$$

$$(3/2)X = 300 \text{ cc} - RA$$

$$X = (2/3)(300 \text{ cc} - RA)$$

$$X = 200 \text{ cc} - (2/3)RA$$

Since there is no room air during the first 300 cc of tidal alveolar ventilation, RA = 0, thus:

$$X = 200 \text{ cc}$$

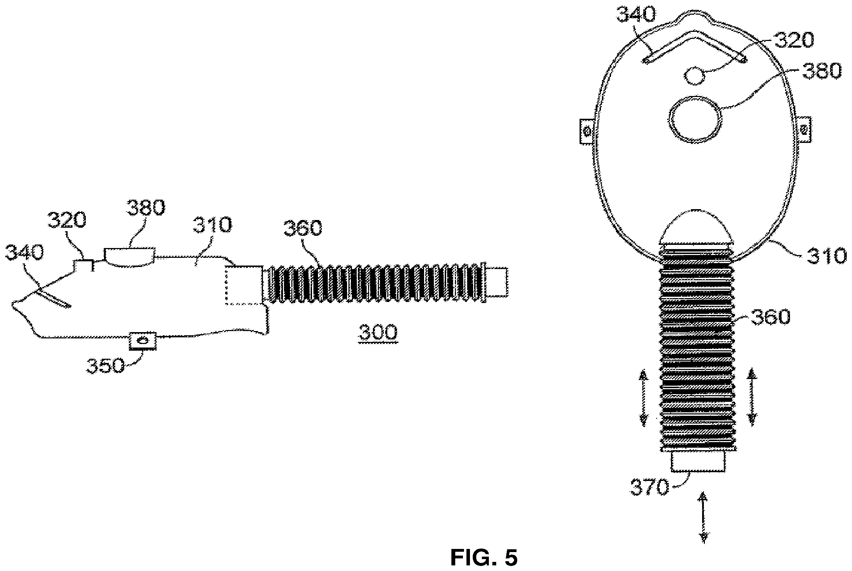

Therefore, for a final volume of 300 cc delivered to the alveolar in the first half of inspiration, the total reservoir volume is approximately 200 cc. As the total reservoir volume is approximately 200 cc, if the oxygen mask of the present disclosure can itself store 150 cc, then any attached storage structures (such as a connected storage reservoir tube) can be adjusted to provide an additional 50 cc of storage volume for a total reservoir volume of 200 cc. With the total reservoir volume, the appropriate flow rate can be calculated. Since the flow rate per respiratory phase is 200 cc/phase and there are three respiratory phases (i.e., inspiration, expiration, expiratory pause), the flow rate per respiratory cycle is 600 cc/respiratory cycle (200 cc/phase x 3 phases/cycle = 600 cc/cycle). With a respiratory rate ("RR") of 20 respiratory cycles per minute, the flow rate is 12L/minute (600 cc/cycle x 20 cycles/minute = 12,000 cc/minute). Thus, if the oxygen mask of the present disclosure is adjusted to have a total reservoir volume of 200 cc and the flow rate is adjusted to 12L/minute, then 200 cc of 100% oxygen stored in the reservoir (and any connected storage reservoir tubes) along with 100 cc of 100% oxygen from the continuously flowing oxygen source will lead to a total of 300 cc of 100% oxygen delivered to the alveolar in the first half of inspiration prior to delivery of room air and flowing oxygen mixture, which comprises the remaining amount of the tidal volume (150 cc), for a total tidal volume of 450 cc. As this embodiment utilizes a flow rate of 12L/minute, a higher $FiO_2$ mask can be used such as the embodiment shown in FIG. 5. The above equations and calculations can be used to determine the appropriate total reservoir volume and flow rate.

8. Improvement of efficacy of alveolar oxygenation by employing a Sequential Gas Delivery ("SGD") pattern:

On inspiration, the oxygen that was saved or collected in the reservoir space during the expiratory pause, in addition to the continuously flowing oxygen provided to the mask, will be delivered to the alveoli in a SGD pattern. In this manner, 100% oxygen is first delivered, without dilution with room air or expired gases from dead space ventilation, at the most effective and efficient oxygen delivery time, PIFR time. In many cases, this time frame is the first about 0.2 to about 0.3 seconds of the early part of inspiration. This initial oxygen delivery is followed by make-up tidal volume which can comprise oxygen from the continuous oxygen flow with room air.

Thus, an oxygen mask of the present disclosure defines a reservoir for storing oxygen. According to an embodiment, the reservoir varies from approximately 20 cc to 200 cc. In an embodiment, the reservoir is approximately 20 cc to 100 cc or 75 cc to 150 cc. In a particular embodiment, the reservoir is approximately 75 cc. Volume can be changed depending on the design of the structure and the function of the mask for various different clinical applications. Oxygen is stored during expiratory pause is delivered as a bolus (approximately 20-150 cc out of 450 cc of normal breath volume) during inspiration. This early 20-150 cc of bolus of oxygen rich air will be inhaled to the alveolar effectively and efficiently where oxygen is exchanged at the most effective and efficient time for alveolar oxygenation.

In use, the oxygen mask of the present disclosure can be used in combination with nasal cannulation whereby nasal cannula can be used as an oxygen flow source with or without end tidal $CO_2$ sampling and the oxygen mask of the present disclosure can function as a reservoir. Unlike conventional oxygen masks, the oxygen masks of the present disclosure are operative to define a reservoir which collects, holds and makes immediately available an oxygen enriched reservoir. This reservoir can be immediately adjacent the patient's airway. Oxygen in this reservoir can be immediately inhaled as the patient breathes. In this regard, oxygen continuously fed via nasal cannula will fill the reservoir defined by the mask during such time as the patient pauses between taking breaths.

The oxygen reservoir thus not only defines a reservoir of oxygen, it also selectively positions the oxygen directly about the patient's airway so that the patient can and must breath in the oxygen enriched air versus air where the supplemented oxygen is either diluted and/or is dispersed away from the nose and mouth of the patient when the patient uptakes air. In this regard, the oxygen mask of the present disclosure can maintain the same bolus that is readily accessible for every breath the patient takes in.

The oxygen mask of the present disclosure can further deliver auto-adjusted boluses of back-up oxygen to accommodate missed or shallow breaths by patients with "nocturnal oxygen desaturation", which is a common and important issue among COPD patients in long-term oxygen therapy.

This is in contrast to conventional oxygen masks that deploy a constant flow of oxygen and air, only a portion of which is actually consumed at select moments (e.g., first 2/3 of first half of inspiration) when the patient breathes. For example, with a standard nasal cannula and conventional oxygen mask, the continuous flow of oxygen during patient exhalation is wasted to the atmosphere. However, the reservoir defined by the oxygen mask of the present disclosure is designed to store approximately 20 cc and up to 150 cc of oxygen. The storage volume depends on factors including, but not limited to, the respiratory rate of the patient and minute alveolar ventilation. This feature helps reduce the effect of dilution/dispersion of gas by entrained room air at normal inspiratory rates. By virtue of the increased efficiency, as well as the subsequent benefits of more thorough oxygenation to the patient, a substantially higher degree of care can be provided.

Conventional masks containing a reservoir cannot achieve the above. For example, conventional masks with three one-way valves and an attached reservoir bag cannot achieve the above at least because this type of mask cannot effectively remove expired gases within the body of the mask prior to the next inspiration. Thus, upon inspiration, this conventional mask cannot deliver 100% of oxygen first using the SGD pattern as exhaled dead space air will be inhaled first instead of 100% oxygen. This conventional mask and its attachments create mechanical dead space, which makes this mask act as a rebreathing system, limiting its use in certain clinical applications. Because dead space will affect alveolar oxygenation (with expired gases) when the tidal volume or fresh gas flow is lower than normal, this conventional mask has limited use with small-sized patients (who have lower tidal volumes). Additionally, the maximum $FiO_2$ of this conventional mask is limited to be lower than the $FiO_2$ levels the oxygen mask of the present disclosure can achieve regardless of whether a higher oxygen flow rate is used.

9. Improved control of the dosage of oxygen (or other gas or drug) delivered to the patient:

By adjusting the volume of oxygen stored in the reservoir during expiratory phase, the oxygen mask of the present disclosure can more accurately determine oxygen delivered to the alveolar. In doing so, the oxygen mask of the present disclosure can deliver constant performance that is independent of the patient factor PIFR and total expiratory time. The oxygen mask of the present disclosure serves as a reservoir during the expiratory pause and the oxygen delivered is not diluted with atmospheric air prior to entering the respiratory tract. By storing a known quantity of oxygen in the oxygen mask of the present disclosure ahead of inspiration, and by delivering stored oxygen without dilution from atmospheric air prior to delivery to the lung, the oxygen mask of the present disclosure enables delivery of a known quantity of oxygen to the alveolar. Importantly, this allows the oxygen mask of the present disclosure to not be limited to high flow rates to overcome room air entrainment from PIFR effect during inspiration, like seen with conventional oxygen masks such as the Ventimask.

Control of oxygen dosage is critical for several reasons. First, accurate delivery of a controlled dosage of oxygen is beneficial to patients with certain conditions. For some conditions, a lack of control of the dosage of oxygen could be injurious or even fatal by delivering insufficient or too much oxygen or delivering oxygen in inconsistent dosages. For example, patients with chronic obstructive pulmonary disease ("COPD") commonly require oxygen treatment because they generally suffer from insufficient oxygen flow into the alveolar. In another example, many emergency patients require 100% oxygen delivery to stabilize the patients. Since COPD patients and many emergency patients typically require certain dosages of oxygen to stabilize, insufficient oxygen delivery caused by a lack of control of oxygen can further exacerbate the COPD patients' or emergency patients' medical status. Thus, the oxygen mask of the present disclosure can more effectively stabilize such patients by accurately and consistently delivering the necessary dosage of oxygen.

Other benefits of improved dosage control include: more definable and predictable alveolar oxygen concentration; standardized device performance; consistent device performance; safer and more effective usage of device; less confusion for end user; device more capable for repeat assessment for therapeutic response; and improved diagnostic and prognostic value.

10. Improved safety through 100% (or near 100%) $FiO_2$ delivery with lower oxygen flow:

Delivering 100% (or near 100%) $FiO_2$ can be important for many patients such as stroke victims, cardiac arrest victims, and other patients with diseases characterized by hypoxic conditions at the emergency site or scene until medical team care is available (e.g., ambulance pre-hospital care from the site to the hospital). Many emergency care vehicles, such as ambulances, carry conventional oxygen devices, such as the non-rebreather mask, which can deliver approximately 60% $FiO_2$, far lower than 100%. The conventional non-rebreather mask is the most widely recognized and utilized oxygen mask in healthcare and was designed to deliver maximum oxygen concentrations. The non-rebreather mask was further designed with one-way valves to direct gas from an oxygen reservoir into the mask during inhalation. During exhalation, two one-way valves located on the sides of the mask open to release expired gas into the atmosphere.

For the patient's safety concern in the event when the source of oxygen was interrupted or disconnected, one of the exhalation valves of the conventional non-rebreather mask is commonly removed to protect from the suffocation. This removal of one exhalation valve is responsible for continuous room air entrainment during inspiration. The conventional non-rebreather mask therefore delivers significantly lower oxygen concentration than 100% despite being designed for patients in acute or critical episodes of distress. The results of using sub-therapeutic oxygen levels include further deterioration of the patient's condition leading to application of more advanced therapeutic interventions. Remedying such inefficient oxygen delivery devices includes treatment using additional procedures, mechanical ventilation with endotracheal intubation. Not only do these treatments present safety risk to the patient but also incur unnecessary costs and prolong hospital stays.

The oxygen mask of the present disclosure improves safety for patients and reduces or eliminates the risk factors of conventional non-rebreather masks related to inefficient oxygen delivery by delivering 100% (or near 100%) $FiO_2$ even with lower oxygen flows than typically used for conventional masks.

11. Improved preservation of natural water vapor:

Water vapor can be collected and stored in a reservoir at least because the oxygen mask of the present disclosure can use a lower flow of oxygen. Benefits of preserving water vapor include preventing dry mouth, mucous plugging, and nasal mucosa drying.

12. Reduction of costs in producing the oxygen mask:

Conventional masks can use multiple one-way valves and/or reservoir bags that can result increased costs of production. The oxygen masks of the present disclosure can provide a full range of oxygen masks, including inexpensive masks. By not utilizing one-way valves and/or reservoir bags, oxygen masks of the present disclosure can reduce production costs. However, some oxygen masks of the present disclosure may incorporate one-way valves and/or reservoir bags.

Additionally, the oxygen mask of the present disclosure can decrease healthcare costs of patients by improving safety and preventing additional negative health events due to inefficient oxygen delivery.

The fundamental principles of the oxygen mask of the present disclosure can be utilized in a wide variety of applications where more efficient delivery of oxygen to a patient is desired (for example, for better safety or effectiveness).

Exemplary embodiments will now be further described with reference to the Drawings.

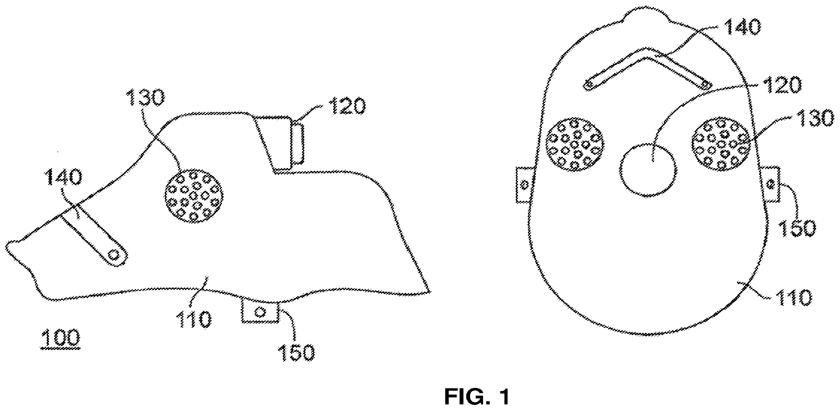

Referring now to FIG. 1, a conventional oxygen mask, shown generally as reference number **100**, is illustrated. As shown, conventional oxygen mask **100** comprises the following elements: a mask body **110**, an inlet port **120**, vent ports **130**, a nose clip **140**, and strap attachments **150**. In conventional oxygen masks, the vent ports are generally formed near the nasal area of the conventional oxygen masks. For example, in the illustrated conventional oxygen mask, the vent ports **130** are formed on the upper half of the mask body **110**. When the conventional oxygen mask **100** is worn, the vent **ports130** will be near the nasal area, oral area, or both.

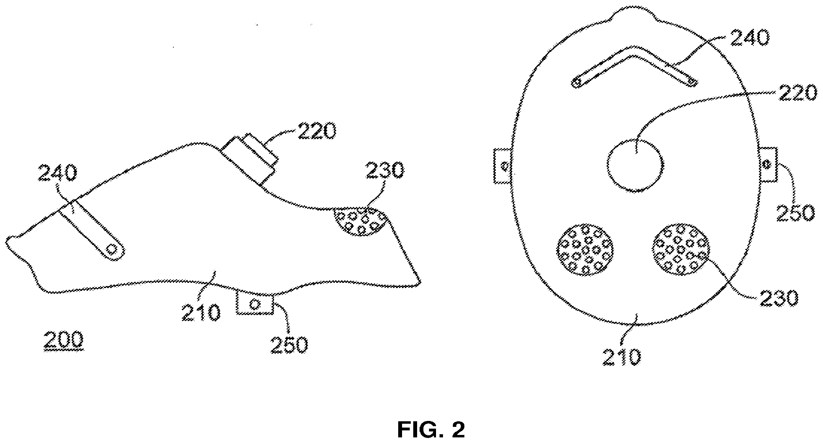

FIGS. 2-7 illustrate oxygen masks embodying one or more aspects of the present disclosure. In general, the oxygen masks of the present disclosure can be lightweight, comfortable to wear, ergonomically shaped, and/or disposable.

FIG. 2 illustrates an embodiment where the oxygen mask **200** includes a mask body **210**, an oxygen port **220**, vent ports **230**, a nose clip **240**, and strap attachments **250**.

The generally concave mask body **210** is molded of a generally gas-impermeable material, such as non-toxic medical grade plastic polymer material. In an embodiment, the mask body **210** is made of silicone or polyvinyl chloride. The mask body **210** material can be transparent to allow clinicians to observe the patient's condition. The mask body **210** and connections and attachments thereto may be disposable.

The mask body **210** defines a cavity adapted to fit over the mouth and the nose of the patient. The peripheral edge of the mask body **210** is contoured so as to substantially seal against the surrounding facial tissue of the patient, to establish an inner chamber portion or inner-space. The peripheral edge can be any shape as long as it is contoured so as to substantially seal against the surrounding facial tissue of the patient. The peripheral edge may be formed to include one or more recesses to allow nasal cannula to access the inner-space.

In various embodiments, the mask body further comprises an interface (not pictured) which can be attached to the peripheral edge of the mask body **210** to inhibit an inner-space of the mask body from substantial contamination with room air when the oxygen mask is in use. The interface can be made of various materials including, but not limited to, cushion, padding, foam, and elastic.

The volume of the inner-space in accordance with present disclosure is generally smaller than those of conventional masks. The mask **200** is very efficient in delivering oxygen and thus does not require as much inner-space for oxygen storage (e.g., a reservoir) as required for conventional masks. In certain embodiments, the volume of the inner-space is about 25 to about 100 cc. Accordingly, the oxygen mask **200** can be configured to store about 25 to about 100 cc of oxygen when the oxygen mask **200** is worn. In a particular embodiment, the oxygen mask **200** is configured to store about 75 cc of oxygen when the oxygen mask **200** is worn.

The oxygen port **220** is configured to accept a standard oxygen tube. In various embodiments, the oxygen port **220** can be configured to accept any type of oxygen tube. The oxygen port **220** can be configured to accept any combination of gas or drug with oxygen. The oxygen port **220** can be configured to accept a $CO_2$ sample line with oxygen tube. The oxygen port **220** allows oxygen to flow from an oxygen source (not pictured) to the inner-space of the oxygen mask **200**. The oxygen port **220** is formed on the upper half of the mask body **210**. In an embodiment, the oxygen port **220** is formed right above the half-way line that separates the upper half and the bottom half of the mask body **220**. In this manner, the oxygen port **220** is located around the nostrils of the patient when the oxygen mask **200** is worn allowing oxygen to be delivered to the nasal area.

One or more vent ports **230** are disposed on the bottom half of the mask body **210**, allowing gases to be discharged from the oxygen mask. In various embodiments, the vent port(s) **230** are located between one and four inches from the bottom of the mask body **210**, around three inches from the bottom of the mask body **210**, or around two inches from the bottom of the mask body **210**. In a particular embodiment, the oxygen mask **200** includes two vent ports **230**. The vent ports **230** are disposed on opposite sides of the mask body **210**. The vent ports are located on the mask body **210** such that they are below the patient's mouth when the oxygen mask **200** is worn.

The vent port(s) **230** may include an open port allowing free flow without any resistance to breathe. The vent port(s) **230** may be formed integrally with the molded mask body **210** or may be inserted into the mask body **210.** The vent port(s) are attachable or connectable as accessories in various ways, for example, filter or/and gas valves (PEEP) or one-way valve, etc.

The oxygen mask **200** may have an adjustable nose clip **240** disposed on the upper half of the mask body **210**. In an embodiment, the adjustable nose clip **240** is disposed on the upper half of the mask body **210** such that the nose clip **240** is over the nose when the oxygen mask **200** is worn. The adjustable nose clip **240** can be adjusted to conform to the nose to stabilize the oxygen mask **200** in position on the patient's face. The adjustable nose clip **240** can be made of various flexible materials. In an embodiment, the adjustable nose clip **240** is made of metal.

The oxygen mask can include any number of strap attachments **250**. In the illustrated embodiment, the oxygen mask includes two strap attachments **250**. The two strap attachments **250** are disposed on opposing sides of the mask body **210** and are integrally molded with the mask body. In various embodiments, the strap attachments **250** can be separate pieces from the mask body and attached to the mask body through different means (e.g., adhesive, fasteners, and screws). The strap attachments **250** can include holes disposed thereon to allow straps to be attached to the strap attachments **250**.

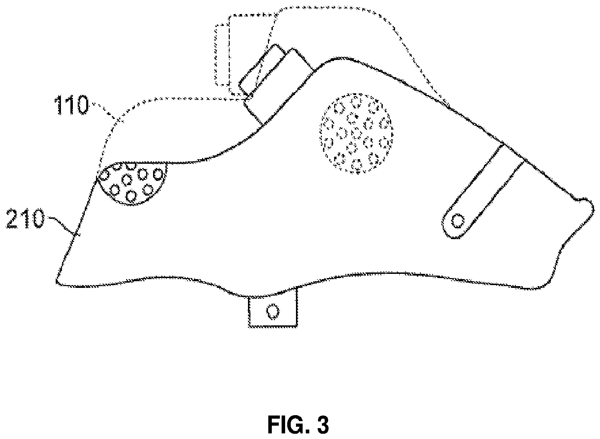

FIG. 3 shows a comparison of the conventional oxygen mask **100** shown in FIG. 1 and the oxygen mask **200** shown in FIG. 2. The conventional oxygen mask **100** is shown as the dashed lines, while the oxygen mask **200** is shown as the solid lines. Generally, the mask body **210** has a slimmer profile than the mask body **110** because the mask **200** does not need an much inner-space as that of the mask **100** due to its higher efficiency in oxygen delivery. The vent ports are placed differently in the two oxygen masks: the vent port(s) of oxygen mask **200** is disposed near the bottom of the mask on the mask body **210**; whereas, the vent port(s) of the conventional oxygen mask **100** is disposed near the nasal area (or the central area) of the mask body **110**.

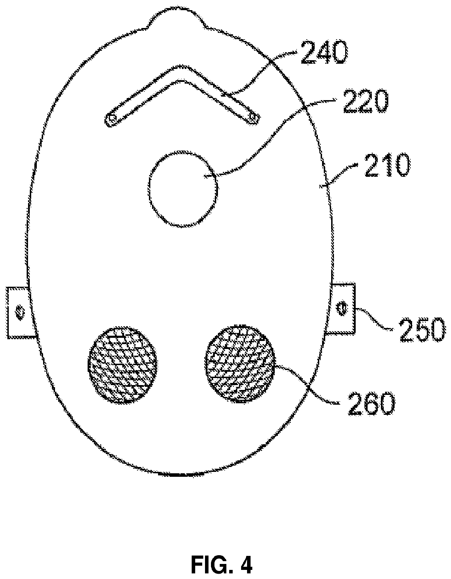

FIG. 4 illustrates another embodiment of the oxygen mask of FIG. 2. The oxygen mask **200** can optionally include filters **260**, which can be placed over each vent port **230** or can be disposed on oxygen mask **200** instead of vent ports **230**. The filters **260** are designed to filter out various undesirable materials (such as pathogens). The filters **260** can each be any shape or size in order to optimize filtering while eliminating (or reducing) resistance to breathe.

FIG. 5 describes an oxygen mask according to the present invention, shown as reference number 300, with exception of the annular aperture. The oxygen mask **300** of the present invention includes a mask body **310**, oxygen storage tube **360**, vent port **370**, oxygen port 380 and annular aperture 560 (not shown in Fig. 5), and can further include supplemental oxygen port 320, nose clip 340 and strap attachments 350. Nose clip **340** and strap attachments **350** are in accordance with other embodiments of the present disclosure.

The mask body **310** is as described above with the mask body **210**.

The oxygen port **380** is as described above with the oxygen port **220**.

The supplemental oxygen port **320** is formed near the central area or upper half of the mask body **310**. The supplemental oxygen port **320** can be configured to allow an oxygen source (not pictured) to connect to the supplemental oxygen port **320**. In this manner, oxygen can be delivered via both the supplemental oxygen port **320** and oxygen port **380** to provide a higher $O_2$ concentration than oxygen were delivered only by oxygen port **380**. The placement of the supplemental oxygen port **320** can allow oxygen to be directly delivered to the inner-space or carbon dioxide to be directly sampled from the inner-space. In an embodiment, the supplemental oxygen port **320** is formed above the halfway line that divides the upper half and bottom half of the mask body **310** and right above the oxygen port **380**. The supplemental oxygen port **320** can be any shape (e.g., generally circular, generally oval-shaped, generally rectangular). The size of the supplemental oxygen port **320** can be between 0.1 inches and 3 inches in diameter (or by longest length). In an embodiment, the supplemental oxygen port **320** is circular in shape and about 1 inch in diameter. In an embodiment, the oxygen port **380** can be configured as an aerosol therapy port to allow aerosol units or Heliox (or other gas) therapy port to be connected to the oxygen port **380**. When oxygen port **380** is used for aerosol therapy, oxygen supplemental port **320** can be used to deliver additionally needed oxygen directly to the inner-space.

The filter, Positive End-Expiratory Pressure (PEEP), one-way valve or other application parts can be applied (attached) between mask body **310** and the oxygen storage tube **360** or at the end of the oxygen storage tube near vent port **370**. The oxygen storage tube **360** may be formed integrally on the molded mask body **310** or may be attached to the mask body **310** through a connection hole in the mask body **310**. The oxygen storage tube **360** is formed on or connected to the mask body **310** near the bottom of the mask body **310**, for example, within three inches from the bottom of the mask body **310**. In the illustrated embodiment, the oxygen storage tube **360** is attached to the mask body **310** within one inch from the bottom of the mask body **310**. The oxygen storage tube **360** is adjustable and can be made of any gas-impermeable material, such as non-toxic medical grade plastic polymer material (e.g., silicone or polyvinyl chloride).

According to the present invention, the oxygen storage tube **360** is corrugated as shown to allow adjustment of the length. By adjusting the length of the oxygen storage tube **360**, the volume of the oxygen storage tube **360** can be increased and decreased. In an embodiment, the total oxygen storage volume is mask body **310** volume plus oxygen storage **360** volume. The total oxygen storage volume is configured to have a volume correlating to about 2/3 the alveolar minute ventilation of the patient. The diameter of the oxygen storage tube **360** can be from about 0.5 inches to about 2.0 inches, or from about 0.75 inches to about 1.0 inches. The length of the fully-extended oxygen storage tube **360** can be from about 1 inch to about 36 inches, or from about 4 inches to about 16 inches. The length (and volume) of the oxygen storage tube **360** can be adjusted according to mask body volume, 2/3 of tidal volume from spirometry values. In the illustrated embodiment, the fully-extended oxygen storage tube **360** is about 12 inches in length.

The distal end of the oxygen storage tube **360** (opposed to the proximal end on the mask body **310**) can include the vent port **370**, allowing gases to flow from the mask body **310** through oxygen storage tube **360** to the outer environment and from the outer environment to oxygen body **310** through oxygen storage tube **360**. In an embodiment, the distal port of the oxygen storage tube **360** has a simple opening as vent port **370**. The location of the vent port **370** on the distal end of the oxygen storage tube **360** allows the vent port **370** to be located further away from the nasal and oral areas of the patient than the oxygen mask **200** illustrated in FIGS. 2-4. The vent port **370** may include perforations. The vent port **370** may be formed integrally with the oxygen storage tube **360** or may be inserted into the oxygen storage tube **360**. The diameter of vent port **370** can be smaller than that of oxygen storage tube **360** to generate back pressure toward alveolar to improve oxygenation from peak expiratory flow rate without increase a resistance to breathe.

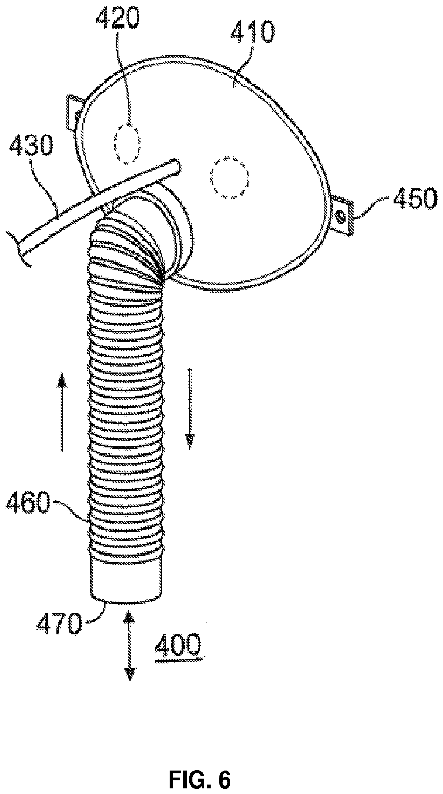

FIG. 6 provides another embodiment of an oxygen mask of the present disclosure. The oxygen mask **400** can be a nasal oxygen conserving mask. The oxygen mask **400** can include a mask body **410**, oxygen port to allow an oxygen supply **430** to deliver oxygen or other gases to inside of the mask body **410**, strap attachments **450**, oxygen storage tube **460**, and vent port **470**. Strap attachments **450**, oxygen storage tube **460**, and vent port **470** are in accordance with other embodiments of the present disclosure. The oxygen mask **400** can optionally include a nose clip (not shown) in accordance with other embodiments of the present disclosure.

In an embodiment, the oxygen mask **400** is shaped to cover the nose of the patient without covering the mouth of the patient. As such, the oxygen supply **430** can be inserted into the oxygen port or dual port for oxygen and sampling port for End Tidal $CO_2$ monitor located near the nasal area and in between the patients' nostrils. Oxygen storage tube **460** can also be located below the oxygen port and below the nostrils (when worn by the patient) to allow expired gases from the nostrils to exit the oxygen mask **400** through the vent port **470**.

Oxygen is supplied to the oxygen mask **400** in various ways known in the art. For example, oxygen can by supplied either continuously (i.e., Continuous Flow Oxygen or "CFO" system) or periodically (such as pulsing). In certain embodiments, a bolus of oxygen is provided periodically to the mask where the amount of the oxygen is determined based on patient's medical need. While the amount can be determined or adjusted by a medical personal based on patient's condition, in certain embodiments, the amount of the bolus of oxygen is about 15 cc to about 25 cc. In other embodiments, the amount of the bolus of oxygen can be lower than 15 cc or greater than 25 cc. The mask body **410** can form an inner space to store a bolus of oxygen (or other gases) in the inner space of the oxygen mask **400** prior to inspiration by the patient. Since inner space of mask body 410 is not enough spaces for the amount of bolus (pulsing) of oxygen, oxygen storage tube 460 is always needed to increase the total reservoir volume of nasal mask.

The oxygen mask **400** can utilize at least two oxygen delivery systems to create the bolus of oxygen in the inner space of the mask body **410**. In one system, the oxygen is pulsed into the inner space of the oxygen mask **410** via the oxygen supply **430**. This can allow for an efficient delivery of oxygen by pulsing the oxygen during the expiratory pause, during which the oxygen is delivered to the inner space of the mask body **410** shortly prior to inspiration. The pulsing oxygen system can be also be used with other embodiments of the present disclosure. In another system, the oxygen is continuously delivered to the inner space of the oxygen mask **410** in accordance with other embodiments of the present disclosure.

In another embodiment, there is provided a nasal pillow mask comprising one or two nasal pillow plugs configured to be connected to an oxygen source. The nasal pillow mask can include any conventional nasal pillow plugs known in the art, and a medical personal can determine a suitable one based on the patient's condition. Each nasal pillow plug can be further configured to fit into a nostril of the patient to allow oxygen to be delivered directly to the nostrils.

The nasal pillow mask comprises one or more reservoir tubes comprising a vent port, typically one or two reservoir tubes. In an embodiment, the mask contains only one reservoir tube which is placed near the nostril area of the user when it is worn. In another embodiment, the mask contains two reservoir tubes, each of which is placed each side, i.e., left-hand and right-hand sides of the mask. In an embodiment, a vent port is placed at the distal end of the reservoir tube. The reservoir tubes can be configured to be adjustable in volume so that a user or medical professional can change the volume of the reservoirs as needed. Also, different types of reservoir tubes can be applied in accordance with required applications, e.g., an oxygen conserving device for long term oxygen therapy for COPD, fixed $FiO_2$ therapy, nebulizer therapy, etc. A person having ordinary skill can determine the size, shape and design of the reservoir tube depending on the user's medical conditions. In a particular embodiment, the reservoir tube is extendable, corrugated tube.

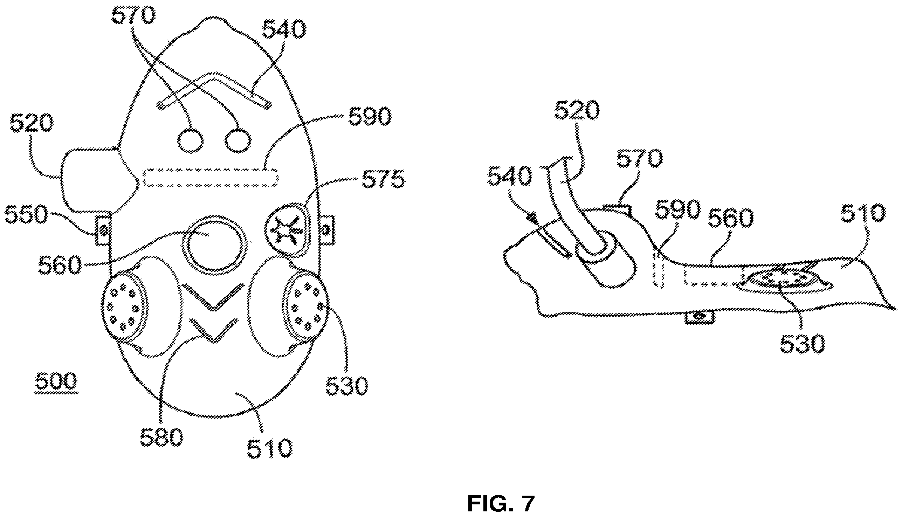

Now referring to FIG. 7, another oxygen mask of the present disclosure is shown as reference number **500**. The oxygen mask **500** can include a mask body **510**, oxygen port **520**, vent ports **530**, nose clip **540**, strap attachments **550**, oral aperture **560**, nasal instrument ports **570**, suction port **575**, flexibility slits **580**, and protrusion **590**. The nose clip **540** and strap attachments **550** are in accordance with other embodiments of the present disclosure.

The generally concave mask body **510** is molded of a generally gas-impermeable material, such as non-toxic medical grade plastic polymer material. In an embodiment, mask body **510** is made of silicone or polyvinyl chloride. The mask body **510** material can be transparent to allow clinicians to observe the patient's condition. The mask body **510** and connections and attachments thereto may be disposable.

The mask body **510** defines a cavity adapted to fit over the mouth and the nose of the patient. The mask body **510** optionally includes a protrusion formed on the inner-space of the mask. In an embodiment, the protrusion **590** can extend from the inner surface of the mask body to create a partial divider. The protrusion **590** is formed on the mask body **510** at a location such that the protrusion **590** at least partially separates the patient's nose and mouth. Thus, the protrusion **590** can be formed between the placement of oral aperture **560** and the top of the mask body **510**. The protrusion **590** can be of various lengths, for example, between about 0.1 inches to about 3 inches. In the illustrated embodiment, the protrusion **590** is about 1.5 inches.

The peripheral edge of the mask body **510** is as described above with the peripheral edge of the mask body **210**.

In various embodiments, the oxygen port **520** can be configured to accept any type of oxygen tube. In an embodiment, the oxygen port **520** is a Luer-lock port. The oxygen port **520** allows oxygen to flow from an oxygen source (not pictured) to the inner-space of the oxygen mask **500**. The oxygen port **520** is formed on the upper half of the mask body **510**, for example, on either side of the mask body **520**. In this manner, the oxygen port **520** is located to the side of the nostrils of the patient when the oxygen mask **500** is worn allowing oxygen to be delivered to the nasal area. In a particular embodiment, the oxygen port **520** is formed on the left side of the mask (i.e., the right side of the patient). Additionally, as a Luer-lock port, the oxygen port **520** can also sample carbon dioxide using a catheter. Thus, respiration (e.g., $CO_2$ waveform) can be monitored for apneic conditions including conditions that could lead to fatal hypoxia.

One or more vent ports **530** are disposed on the bottom half of the mask body **510**, allowing gases to be discharged from the oxygen mask. In various embodiments, the vent port(s) **530** can be located between one and four inches from the bottom of the mask body **510**, around three inches from the bottom of the mask body **510**, or around two inches from the bottom of the mask body **510**. In a particular embodiment, the oxygen mask **500** includes two vent ports **530**. The vent ports **530** are disposed on opposite sides of the mask body **510**. The vent ports are located on the mask body **510** such that they are below the patient's mouth when the oxygen mask **500** is worn.

The vent port(s) **530** may include perforations allowing free flowing of gases. The vent port(s) **530** may be formed integrally with the molded mask body **510** or may be inserted into the mask body **510**.

The oral aperture **560** is formed near the central area or upper half of the mask body **560**. When the oxygen mask **500** is worn, the oral aperture **560** is located above the patient's mouth to provide access to the patient's mouth. In this matter, surgical tools can access the patient's mouth through the oral aperture **560**. In an embodiment, the oral aperture **560** is formed right above the halfway line that divides the upper half and bottom half of the mask body **510**. The oral aperture **560** can be any shape (e.g., generally circular, generally oval-shaped, generally rectangular), as long as the oral aperture can be formed on the mask body **510**. The size of the oral aperture **560** can be between 0.1 inches and 3 inches in diameter (or by longest length). In an embodiment, the oral aperture **560** is circular in shape and about 3/4 inch in diameter. An optional insulator (not pictured) can be placed within the oral aperture to inhibit free flow of gas. The optional insulator can be made of any suitable material including, but not limited to, rubber, silicone and foam. The optional insulator has a hole to allow surgical tools to go through the oral aperture **560** and the optional insulator itself. In an embodiment, the optional insulator is a diaphragm with a continuous, perforable membrane.

The oxygen mask **500** can optionally include one or more nasal instrument ports **570** formed on the upper half of the mask body **510**. In an embodiment, the oxygen mask **500** includes two nasal instrument ports, as illustrated in FIG. 6. In another embodiment, the oxygen mask **500** includes one nasal instrument port. Each nasal instrument port **570** is formed on the upper half of the mask body **510** such that each nasal instrument port **570** allows access to the nose and nostrils of the patient when the oxygen mask **500** is worn. In this manner, nasal instruments can access the nose and nostrils of the patient when the oxygen mask **500** is worn. Similar to the oral aperture **560**, the nasal instrument port **570** can have an optional insulator. In an embodiment, the optional insulator is a diaphragm with a continuous, perforable membrane.

The oxygen mask **500** can optionally include one or more suction ports **575** formed on the mask body **510**. In an embodiment, the oxygen mask **500** includes one suction port **575**, as illustrated in FIG. 6. Generally, the suction port **575** is formed towards the side of the central area of the oxygen mask **500**. The suction port **575** is configured to allow a suction device (not picture) to be attached to the suction port **575**. In this manner, the suction device can remove liquids or other materials from the inner-space. The suction port **575** can be formed in any manner well known in the art. In an embodiment, the suction port **575** includes a diaphragm with a continuous, perforable membrane.

The oxygen mask **500** can optionally include one or more flexibility slits 580 formed on the bottom half of the mask body **510**. In an embodiment, the oxygen mask **500** includes two flexibility slits **580**, as illustrated in FIG. 6, but the number of slits is not critical. Therefore, a person having ordinary skill can determine the number as needed. The flexibility slits **580** can be in any shape (e.g., straight line, curved line, and V-shape). In an embodiment, the flexibility slits **580** are V-shaped. The flexibility slits **580** are formed on the bottom half of the mask body **510** below. For example, the flexibility slits **580** are formed below the oral aperture, as in the illustrated embodiment. The flexibility slits **580** can allow the mask body **510** to be more flexible.

In an embodiment, there is provided an infection prevention mask which can be used in various medical procedures, for example, upper endoscopy. Generally, the mask body is made of a material known in the art that can prevent infection, such as microfiber fabric. The mask body is not transparent except for a window area.

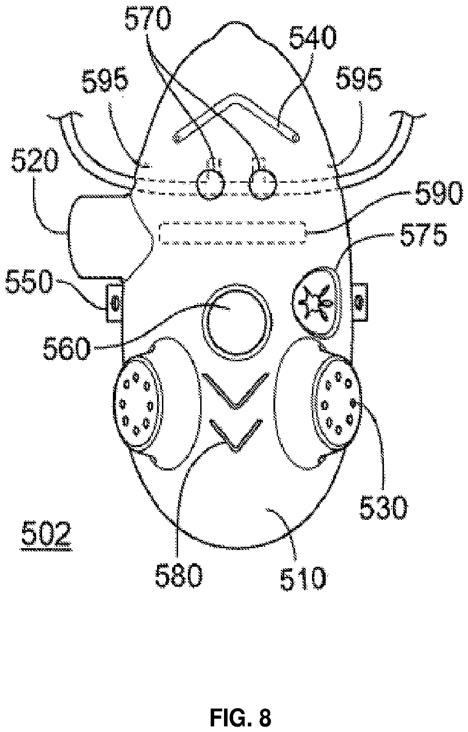

FIG. 8 illustrates another embodiment of the oxygen mask of FIG. 7. The re optional recesses **595** formed on the peripheral edge of the mask body **510**. The recesses **595** are configured and/or shaped to allow nasal cannula to directly access the nose area of the inner-space while minimizing leakage of the inner-space to the outside environment. The recess(s) **595** is/are located on the upper half of the mask body **510**. The recess(s) **595** can be located between one and four inches from the bottom of the mask body **510**, around three inches from the bottom of the mask body **510**, or around two inches from the bottom of the mask body **510**. In an embodiment, the oxygen mask **500** includes two recesses **595**. The recesses **595** are disposed on opposite sides of the mask body **510**. In an embodiment, an oxygen source (not pictured) connected to the nasal cannula can alone supply oxygen to the patient. In an embodiment, an oxygen source (not pictured) connected to the nasal cannula, on conjunction with the oxygen source (not pictured) connected to oxygen port **520**, can supply oxygen to the patient. In an embodiment, the aperture **560** can include a rubber insulation and can further be surrounded by transparent material, such as plastic polymer, to allow viewing of the inner space of the oxygen mask of the present disclosure through the transparent material. In an embodiment, a cushion or other sealing structure can be included along the edges of the oxygen mask of the present disclosure for sealing the mask to the face of the patient and for preventing air leakage.

In an embodiment, the oxygen mask of the present disclosure can be used with nasal cannula instead of an oxygen source connected through the oxygen port **520**. In this embodiment, the oxygen mask of the present disclosure does not include oxygen port **520** and oxygen (or other drugs) are delivered to the patient solely through nasal cannula, which enter the inner space of the mask through recesses **595**. In this manner, the oxygen mask of the present disclosure can simply applied to a patient who already is using nasal cannula, and the oxygen mask can be removed after use, leaving the patient still using the nasal cannula. This can be advantageous for patients undergoing surgical procedures as many patients may require nasal cannula prior to operation and after operation during recovery and the oxygen mask of the present disclosure can be convenient to apply and remove.

EXAMPLE

Example 1: Comparative Studies

In this example, an oxygen mask of the present disclosure is compared against nasal cannula and nasal reservoir cannula (i.e., oxymizer) for how much oxygen is delivered to the alveolar per respiratory cycle and how much oxygen is wasted.

Each respiratory cycle has three phases: inspiration, expiration, and expiratory pause. All three phases have the equal time during the normal spontaneous respiratory cycle. Thus, each phase lasts one third of a normal respiratory cycle. Oxygen reaches the alveolar during the first half of the inspiration phase. 2/3 of the tidal volume reaches alveolar during the first half of the inspiration phase. During other phases of the respiratory cycle, supplied oxygen is generally wasted.

A. Nasal Cannula:

The oxygen flow rate is 4L/minute, while the patient's respiratory rate is 20/minute. Tidal volume is 450 cc. Tidal alveolar ventilation is 300 cc. Thus, the oxygen flow rate/respiratory cycle is about 200 cc (4000cc/20 respiratory rate) and the oxygen flow rate/phase is about 66.6 cc (200/3 cc). During inspiration, about 33.3 cc of 100% oxygen reaches the alveolar and 56 cc of 100% oxygen from 21 % of oxygen of 267 cc of room air (300 cc - 33.3 cc = 266.7 cc and 266.7 x 21/100 = 56cc). Thus, 89 cc of 100% oxygen is delivered to alveolar per breath, which is 30% (89/300 x 100 = 30%). Per minute, about 666.6 cc (33.3 cc x 20 respiratory rate = 666.6 cc) reaches the alveolar out of a total of about 4000 cc of oxygen supplied, resulting in about 17% used and about 83% wasted oxygen. With a 4L/minute flow rate, the fraction of inspired oxygen delivered via standard nasal cannula is about 30% theoretically.

B. Nasal Reservoir Cannula (Oxymizer):

The reservoir cannula is an oxygen conserving device ("OCD"). It can store 20 ml of oxygen during exhalation and makes that oxygen available for the beginning of the next inhalation. The oxygen flow rate is 4L/minute, while the patient's respiratory rate is 20/minute. Tidal volume is 450 cc. Alveolar ventilation is 300 cc. During inspiration, the first half of inspiration time is responsible for alveolar ventilation with about 33.3 cc of oxygen delivered from continuous oxygen flow. A total of about 105 cc of 100% (or near 100%) oxygen is delivered to the alveolar during the first half of inspiration (20 cc from reservoir + 33.3 cc from continuous flow + 52 cc from 21% room air). Per minute, about 1066 cc out of 4000 cc of provided oxygen is used for alveolar oxygenation, resulting in 27% is used and about 73% wasted oxygen. The fraction of inspired oxygen delivered via reservoir nasal cannula is about 35% theoretically.

C. Oxygen Mask of the Present Disclosure:

The sealed oxygen mask saves about 66.6 cc of oxygen during expiratory pause. This about 66 cc bolus of near 100% oxygen along with about 33.3 cc of oxygen from the continuous oxygen flow and 42 cc of oxygen from room air reaches the alveolar during the early part of the inspiration phase for a total of about 141.4 cc of 100% (or near 100%) oxygen per respiration. About 1998 cc of oxygen reaches the alveolar per 4000 cc of oxygen delivered. Thus, about 50% of fresh oxygen from the 4L/minute flow is wasted. The fraction inspired oxygen delivered via Oxygen Mask of Present Disclosure is about 47% theoretically.

Results of this comparatives study is shown in Table 1 below.

Table 1. Comparison of used $O_2$ per 4000 cc supplied $O_2$ and fraction of inspired $O_2$.

| | Used $O_2$ Amount (cc) | Wasted $O_2$ Amount (cc) (%) | FiO$_2$ |
|---|---|---|---|
| Nasal Cannula | 666 | 3333 (83%) | Approx. *34% (**30%) |
| Reservoir Nasal Cannula | 1066 | 2934 (64%) | Approx. *41% (**35%) |
| Oxygen Mask of the Present Disclosure | 1988 | 2012 (50%) | Approx. ***55% (**47%) |
| *Referenced by articles and textbook ** Theoretical calculation *** Measured by End Tidal O2(FeO2) | | | |

The amount of used $O_2$ can be approximated by defining "X" as the amount of oxygen supplied per breathing phase of the respiratory cycle and by using the following equations (with X = 66.6 cc as an example):

$$X = 66.6 \text{ cc of oxygen,}$$

- Nasal Cannula: (1/2)X = 33.3 cc of used oxygen plus room air oxygen,
- Reservoir Nasal Cannula: (1/2)X + 20 cc of reservoir oxygen = 53.3 cc of used oxygen plus room,
- Oxygen Mask of the Present Disclosure: (1/2)X + X = 99.9 cc of used oxygen plus room air oxygen,

The $O_2$ flow that is required to obtain 30% FiO$_2$ with the oxygen mask of the present disclosure can be determined using known TV and RR values. For example, when the TV is 450 cc and RR is 20 cycles/minute, then the total volume delivered during the first half of inspiration is 300 (450 cc x (2/3) = 300 cc). If "Y" represents the amount of 100% oxygen delivered to the alveolar to achieve 30% $FiO_2$, the necessary flow rate can be calculated as follows:

$$100\% \times (Y/300\ cc) = 30\%$$

$$Y \times 100\% = 30\% \times 300\ cc$$

$$Y = 90\ cc$$

Thus, the required amount of 100% oxygen delivered to the alveolar to achieve 30% $FiO_2$ is 90 cc. The oxygen mask of the present disclosure can deliver oxygen according to X + ½X + RA, where X is the amount of 100% oxygen stored in the reservoir during expiratory pause and then delivered to the alveolar during the first half of inspiration, ½X is the amount of 100% oxygen delivered during the first half of inspiration from the continuously flowing oxygen source, and RA is the amount of 100% oxygen from 21% oxygen room air. Thus, the required amount of 100% oxygen stored in the reservoir during expiratory pause can be calculated as follows:

$$X + \tfrac{1}{2}X + RA = 90\ cc\ of\ 100\%\ oxygen$$

$$(3/2)X = 90\ cc\ \text{-}\ RA$$

Since RA is approximately 44 c with 30% $FiO_2$:

$$(3/2)X = 90\ cc - 44\ cc = 46\ cc$$

$$X = (2/3)(46\ cc)$$

$$X = 30.6\ cc$$

With 30.6 cc of 100% oxygen stored in the reservoir during the expiratory pause, 30.6 cc of 100% oxygen is delivered per respiratory phase. Given there are three respiratory phases, 91.8 cc of 100% oxygen is delivered per respiratory cycle (30.6 cc/phase x 3 phases/cycle = 91.8 cc/cycle). With a RR of 20 cycles/min, the required flow rate is 1836 cc/min (91.8 cc/cycle x 20 cycles/minute = 1836 cc/minute). In other words, for this situation, the oxygen mask of the present disclosure requires approximately a flow rate of 1.8L/minute. Given that for 30% $FiO_2$, a nasal cannula requires an oxygen flow rate of 4L/minute, the oxygen mask of the present disclosure saves more than 50% oxygen over the nasal cannula.

The terminology used herein is for the purpose of describing particular example embodiments only and is not intended to be limiting. As used herein, the singular forms "a", "an" and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

**Claims**

1.  An oxygen mask (300) comprising:

    a mask body (310) defining a cavity configured to be positioned over the mouth and nose of a patient,

a corrugated oxygen storage tube (360) directly coupled to a bottom half of the mask body and a vent port (370) formed on the oxygen storage tube (360), wherein the vent port is formed on the oxygen storage tube in a manner that patient's exhaled gases are directed towards the vent port; wherein the length of the oxygen storage tube (360) is adjustable to increase or decrease the storage volume of the oxygen storage tube; and the mask being **characterized by**

- an oxygen port (380) formed on the upper half of the mask body (310) and
- an annular aperture (560) formed on the mask body to provide access to the patient's mouth.

2. The oxygen mask of claim 1, wherein, the total oxygen storage volume of the mask (300) is the oxygen storage volume of the mask body (310) plus the oxygen storage volume of the tube (360)

3. The oxygen mask of claim 1, wherein the oxygen mask is configured to store greater than about 20 cubic centimeters of oxygen when the oxygen mask is worn.

4. The oxygen mask of claim 3, wherein the oxygen mask (300) is configured to store approximately 20 cc to 200 cc of oxygen.

5. The oxygen mask of any one of claims 1 to 4, wherein the distal end of the oxygen storage tube (360) includes the vent port (370), whereby gases flow from the mask body 310 through the oxygen storage tube and whereby the vent port is located further away from nose of the patient than the mask body.

6. The oxygen mask of claim 3, wherein the oxygen storage tube (360) is coupled to a filter .

**Patentansprüche**

1. Sauerstoffmaske (300), umfassend:

einen Maskenkörper (310), der einen Hohlraum definiert, der konfiguriert ist, um über Mund und Nase eines Patienten positioniert zu werden, einen gewellten Sauerstoffspeicherschlauch (360), der direkt mit einer unteren Hälfte des Maskenkörpers gekoppelt ist, und eine Entlüftungsöffnung (370), die an dem Sauerstoffspeicherschlauch (360) gebildet ist, wobei die Entlüftungsöffnung auf eine Weise an dem Sauerstoffspeicherschlauch gebildet ist, dass die ausgeatmeten Gase des Patienten zu der Entlüftungsöffnung geleitet werden; wobei die Länge des Sauerstoffspeicherschlauchs (360) einstellbar ist, um das Speichervolumen des Sauerstoffspeicherschlauchs zu vergrößern oder zu verkleinern; und wobei die Maske **gekennzeichnet ist durch**

- eine Sauerstofföffnung (380), die in der oberen Hälfte des Maskenkörpers (310) gebildet ist, und
- einen ringförmigen Durchlass (560), der an dem Maskenkörper gebildet ist, um einen Zugang zu dem Mund des Patienten bereitzustellen.

2. Sauerstoffmaske nach Anspruch 1, wobei das gesamte Sauerstoffspeichervolumen der Maske (300) das Sauerstoffspeichervolumen des Maskenkörpers (310) plus das Sauerstoffspeichervolumen des Schlauchs (360) ist.

3. Sauerstoffmaske nach Anspruch 1, wobei die Sauerstoffmaske konfiguriert ist, um mehr als etwa 20 Kubikzentimeter Sauerstoff zu speichern, wenn die Sauerstoffmaske getragen wird.

4. Sauerstoffmaske nach Anspruch 3, wobei die Sauerstoffmaske (300) konfiguriert ist, um etwa 20 cm3 bis 200 cm3 Sauerstoff zu speichern.

5. Sauerstoffmaske nach einem der Ansprüche 1 bis 4, wobei das distale Ende des Sauerstoffspeicherschlauchs (360) die Entlüftungsöffnung (370) beinhaltet, wodurch Gase von dem Maskenkörper 310 durch den Sauerstoffspeicherschlauch strömen und wobei die Entlüftungsöffnung weiter von der Nase des Patienten entfernt ist als der Maskenkörper.

6. Sauerstoffmaske nach Anspruch 3, wobei der Sauerstoffspeicherschlauch (360) mit einem Filter gekoppelt ist.

**Revendications**

1. Masque à oxygène (300) comprenant :

   un corps de masque (310) définissant une cavité conçue pour être positionnée sur la bouche et le nez d'un patient, un tube de stockage d'oxygène ondulé (360) directement couplé à la moitié inférieure du corps du masque et un orifice d'aération (370) formé sur le tube de stockage d'oxygène (360), ledit orifice d'aération étant formé sur le tube de stockage d'oxygène de manière à ce que les gaz expirés du patient soient dirigés vers l'orifice d'aération ; la longueur du tube de stockage d'oxygène (360) étant réglable pour augmenter ou diminuer la volume de stockage du tube de stockage d'oxygène ; et le masque étant **caractérisé par**

   - un orifice d'oxygène (380) formé sur la moitié supérieure du corps de masque (310) et
   - une ouverture annulaire (560) formée sur le corps du masque pour donner accès à la bouche du patient.

2. Masque à oxygène selon la revendication 1, le volume de stockage d'oxygène total du masque (300) étant le volume de stockage d'oxygène du corps de masque (310) plus le volume de stockage d'oxygène du tube (360).

3. Masque à oxygène selon la revendication 1, ledit masque à oxygène étant conçu pour stocker plus qu'environ 20 centimètres cubes d'oxygène lors du port du masque à oxygène.

4. Masque à oxygène selon la revendication 3, ledit masque à oxygène (300) étant conçu pour stocker approximativement 20 cc à 200 cc d'oxygène.

5. Masque à oxygène selon l'une quelconque des revendications 1 à 4, l'extrémité distale du tube de stockage d'oxygène (360) comprenant l'orifice d'aération (370), ce qui permet l'écoulement des gaz depuis le corps 310 du masque à travers le tube de stockage d'oxygène et ce qui permet l'implantation de l'orifice d'aération plus loin du nez du patient que le corps du masque.

6. Masque à oxygène selon la revendication 3, ledit tube de stockage d'oxygène (360) étant couplé à un filtre.

FIG. 1

20

**FIG. 2**

**FIG. 3**

FIG. 4

FIG. 5

**FIG. 6**

FIG. 7

**FIG. 8**

## REFERENCES CITED IN THE DESCRIPTION

### Patent documents cited in the description

- US 7004168 B
- US 8960195 B, Lehman

### Non-patent literature cited in the description

- **SHAUL COHEN et al.** TSE 'Mask' Improves Oxygenation in Deeply Sedated Patients with Nasal Cannula during Upper Endoscopy. *Anesthesiology,* 2007, vol. 107, A922
- *Poster Presentation at American Society of Anesthesiologists Annual Meeting,* October 2007
- **LEIGH JM.** Variation in Performance of Oxygen Therapy Devices. *Annals of The Royal College of Surgeons of England.,* 1973, vol. 52 (4), 234-253